(19) Europäisches Patentamt

European Patent Office

Office européen des brevets

(11) Publication number: **0 450 240 A1**

## EUROPEAN PATENT APPLICATION

(21) Application number: 90400938.8

(22) Date of filing: 05.04.90

(51) Int. Cl.5: **A61K 37/02, A61K 37/24**

(43) Date of publication of application:
09.10.91 Bulletin 91/41

(84) Designated Contracting States:
AT BE CH DE DK ES FR GB GR IT LI LU NL SE

(71) Applicant: **Soma, Gen-Ichiro**
**1-10-21, Higashi-Tamagawa**
**Setagaya Ward, Tokyo(JP)**

Applicant: **Mizuno, Den'ichi**
**18 Okamoto**
**Kamakura City, Kanagawa(JP)**

(72) Inventor: **Soma, Gen-Ichiro**
**1-10-21, Higashi-Tamagawa**
**Setagaya Ward, Tokyo(JP)**
Inventor: **Mizuno, Den'ichi**
**Okamoto-18, Kamakura City**
**Kanagawa(JP)**
Inventor: **Tsuji, Yoshiaki, Biotechnology**
**Research Center**
**Teikyo Univ., 1091 Sun Sawa-arashi,**
**Sagamiko-Cho**
**Tsukui-Gun, Kanagawa(JP)**
Inventor: **Kobayashi, Nobuyuki**
**Medical Department of Yamaguchi**
**University**
**Kogushi-1144, Ube City, Yamaguchi(JP)**

(74) Representative: **Ahner, Francis et al**
**CABINET REGIMBEAU, 26, avenue Kléber**
**F-75116 Paris(FR)**

(54) Anti-aids preparation.

(57) An anti-AIDS preparation comprising, as an active agent, a pharmaceutically effective amount of a polypeptide selected from the group consisting of TNF-$\alpha$, TNF$_{AM1}$, TNF$_{AM2}$, Th$\beta_4$-AM1, Th$\beta_4$-AM2, and mixtures of at least two of these Polypeptides, a method of treating AIDS in a mammal, comprising administerring said preparation to a mammal infected with HIV retroviruses, and a method of preventing the reoccurrence of AIDS in a mammal displaying AIDS symptoms comprising administerring said preparation to said mammal for a period of time sufficient to kill substantially all T4 cells of said mammal infected with HIV retrovirus are disclosed herein.

EP 0 450 240 A1

Field of the invention

The present invention relates to anti-AIDS preparation. More particularly, it is concerned with anti-AIDS preparations which treats AIDS on the basis of the system of action where the preparation kills T4 cells infected with an AIDS-causing factor, and/or such factor is excluded from the T4 cells, and further prevents the occurrence of AIDS in the patients infected with such factor.

Description of the Prior Art

AIDS is an abbreviation of acquired immunedeficiency syndrome which is a very new disease the first case of which was confirmed to have occurred in 1981. It has already, however, become the subject of heightened attention and concern. This is particularly the case because most of the patients are of working age, and their generation may be exterminated by cross-infection from the mothers at the foetal stage.

The AIDS-causing factor is human immunodeficiency virus (hereunder referred to as HIV) which is a retrovirus. Though the whole aspect of HIV infection has not been elucidated yet, it is understood that AIDS occurs if the immune system of the patient becomes deficient due to reduced function of T4 cells infected by HIV. Such an individual is frequently at risk from the attack of various secondary infections. Thus, AIDS cannot be perfectly cured unless the HIV-infected cells are destroyed, or the HIVs are excluded from said cells.

Investigations have been made to develop an anti-HIV medicine, and as a result, now HIV reverse transcriptase inhibitors (hereunder referred to as RT inhibitors) which includes azidothymidine (hereunder referred to as AZT) or suramin have been developed. These RT inhibitors prevent a retrovirus genome from being inserted into a chromosomic DNA, so they may be able to prevent the infection of HIVs, or to prevent the HIVs from spreading in the HIV-infected patients. But, these inhibitors cannot exclude the already inserted HIVs, and thus the HIVs become active again, and AIDS condition is aggravated if their use is suspended. In conclusion, the prior art RT inhibitors can neither cure AIDS perfectly nor prevent its occurrence.

The interval between HIV infection and the occurrence of AIDS is typically 4 or more years. Thus, the cases of AIDS patients reported already constitute only a part of forseen patients. That is, it is quite plain that the number and severity of the HIV-infections worldwide will increase sharply in the coming years. Therefore, it is of urgent necessity to develop a medicine capable of curing AIDS and preventing its occurrence in HIV-infected patients by specifically destroying HIV-infected cells or excluding the HIVs from potential infection sites.

BRIEF SUMMARY OF THE INVENTION

Under the circumstances mentioned above, the present invention is intended to provide a novel anti-AIDS preparation which cures AIDS and further prevents its occurrence in HIV-infected patients. The system of the action of the preparation has not been elucidated completely yet, but it is believed that the system of action is based on the specific destruction of the HIV-infected cells or the exclusion of the HIVs from said infected cells.

BRIEF DESCRIPTION OF THE DRAWINGS

Fig. 1 shows a base sequence of the Xho-Pstl fragment of the genome genes of the anti-tumor polypeptide purified from THP-I cells.

Fig. 2 is a chart showing the elution pattern of the $Th\beta_4$-TNF fused protein-containing supernatant from centrifugation observed when the supernatant was subjected to the first mono Q FPLC column chromatography.

Fig. 3 is a chart showing the elution pattern of the active fractions, which were collected in the light of the elution pattern shown in Fig. 2, observed when the fractions subjected to the second mono Q FPLC column chromatography.

Fig. 4 is a graph demonstrating the cytotoxicity of $TNF_{AM2}$ of the present invention to Molt-4/HIV cells using a cell survival rate (%) as an indication.

Fig. 5 is a graph demonstrating the cytotoxicity of $Th\beta_4$-AM1 of the present invention to Molt-4/HIV cells using a cell survival rate (%) as an indication.

Fig. 6 is a graph demonstrating the cytotoxicity Of $Th\beta_4$-AM1 of the present invention to Jurkat/HIV cells using a cell survival rate (%) as an indication.

Fig. 7 is a graph demonstrating the growth rates of various T4 cells observed 3 days after the administration of TNF$_{AM2}$ of the present invention.

Fig. 8 is a graph demonstrating the growth rates of various T4 cells observed 6 days after the administration of TNF$_{AM2}$ of the present invention.

Fig. 9 is a graph demonstrating the cytotoxicity of TNF-$\alpha$ and others of the present invention to Molt-4 cells not infected with HIVs in terms of cell growth rates observed 10 days after the administration.

Fig. 10 is a graph demonstrating the cytotoxicity of TNF-$\alpha$ and others of the present invention to CCRF-CEM cells not infected with HIVs in terms of cell growth rates observed 12 days after the administration.

## DETAILED DESCRIPTION OF THE INVENTION

The novel anti-AIDS preparation of the present invention contains TNF-$\alpha$, TNF$_{AM1}$, TNF$_{AM2}$, certain thymosin $\beta_4$-TNF fused proteins individually or two or more of them in admixture as the main active ingredient(s).

TNF-$\alpha$ is a publicly known polypeptide represented by the following amino acid sequence A:

Met-Val-Arg-Ser-Ser-Ser-Arg-Thr-Pro-Ser-Asp-Lys-Pro-Val-Ala-His-

Val-Val-Ala-Asn-Pro-Gln-Ala-Glu-Gly-Gln-Leu-Gln-Trp-Leu-Asn-Arg-

Arg-Ala-Asn-Ala-Leu-Leu-Ala-Asn-Gly-Val-Glu-Leu-Arg-Asp-Asn-Gln-

Leu-Val-Val-Pro-Ser-Glu-Gly-Leu-Tyr-Leu-Ile-Tyr-Ser-Gln-Val-Leu-

Phe-Lys-Gly-Gln-Gly-Cys-Pro-Ser-Thr-His-Val-Leu-Leu-Thr-His-Thr-

Ile-Ser-Arg-Ile-Ala-Val-Ser-Tyr-Gln-Thr-Lys-Val-Asn-Leu-Leu-Ser-

Ala-Ile-Lys-Ser-Pro-Cys-Gln-Arg-Glu-Thr-Pro-Glu-Gly-Ala-Glu-Ala-

Lys-Pro-Trp-Tyr-Glu-Pro-Ile-Tyr-Leu-Gly-Gly-Val-Phe-Gln-Leu-Glu-

Lys-Gly-Asp-Arg-Leu-Ser-Ala-Glu-Ile-Asn-Arg-Pro-Asp-Tyr-Leu-Asp-

Phe-Ala-Glu-Ser-Gly-Gln-Val-Tyr-Phe-Gly-Ile-Ile-Ala-Leu

TNF$_{AM1}$ and TNF$_{AM2}$ are polypeptides represented by the following amino acid sequence B:

Met-Val-Arg-Ser- X -Thr-Arg-Thr-Pro-Ser-Arg-Lys-Pro-Val-Ala-His-

Val-Val-Ala-Asn-Pro-Gln-Ala-Glu-Gly-Gln-Leu-Gln-Trp-Leu-Asn-Arg-

Arg-Ala-Asn-Ala-Leu-Leu-Ala-Asn-Gly-Val-Glu-Leu-Arg-Asp-Asn-Gln-

Leu-Val-Val-Pro-Ser-Glu-Gly-Leu-Tyr-Leu-Ile-Tyr-Ser-Gln-Val-Leu-

Phe-Lys-Gly-Gln-Gly-Cys-Pro-Ser-Thr-His-Val-Leu-Leu-Thr-His-Thr-

Ile-Ser-Arg-Ile-Ala-Val-Ser-Tyr-Gln-Thr-Lys-Val-Asn-Leu-Leu-Ser-

Ala-Ile-Lys-Ser-Pro-Cys-Gln-Arg-Glu-Thr-Pro-Glu-Gly-Ala-Glu-Ala-

Lys-Pro-Trp-Tyr-Glu-Pro-Ile-Tyr-Leu-Gly-Gly-Val-Phe-Gln-Leu-Glu-

Lys-Gly-Asp-Arg-Leu-Ser-Ala-Glu-Ile-Asn-Arg-Pro-Asp-Tyr-Leu-Asp-

Phe-Ala-Glu-Ser-Gly-Gln-Val-Tyr-Phe-Gly-Ile-Ile-Ala-Leu

wherein TNF$_{AM1}$ corresponds to the case where X is Ser, and TNF$_{AM2}$ corresponds to the case where X is Cys.

In the amino acid sequence B given above, the portion from the Ala located in the 19th site to the last Leu is the same as the amino acid sequence which is constructed by linking guanine (G) with the beginning of the amino acid sequence of the fourth exon of the TNF-α. Accordingly, throughout the specification and the claims, the Ala-Leu portion is expressed by the passage "the amino acid sequence corresponding to the base sequence constructed by linking guanine with the beginning of the base sequence of the fourth exon of TNF-α"

The certain thymosin β₄-TNF fused proteins available for use in the present invention are represented by the following amino acid sequence C:

Ser-Asp-Lys-Pro-Asp-Met-Ala-Glu-Ile-Glu-Lys-Phe-Asp-Lys-Ser-Lys-

Leu-Lys-Lys-Thr-Glu-Thr-Gln-Glu-Lys-Asn-Pro-Leu-Pro-Ser-Lys-Glu-

Thr-Ile-Glu-Gln-Glu-Lys-Gln-Ala-Gly-Glu-Ser-Asp-Pro-X-

(the amino acid sequence corresponding to the base sequence constructed by linking guanine with the beginning of the base sequence of the fourth exon of TNF-α) wherein the X represents either of the following two amino acid sequences:

    1) Val-Arg-Ser-Ser-Thr-Arg-Thr-Pro-Ser-Arg-Lys-Pro-Val-Ala-His-Val-Val; and

    2) Val-Arg-Ser-Cys-Thr-Arg-Thr-Pro-Ser-Arg-Lys-Pro-Val-Ala-His-Val-Val.

In the above amino acid sequence C, the sequence of the first 43 amino acids corresponds to the amino acid sequence of thymosin β₄ (hereunder referred to as Thβ₄) which is reported to revive immunity [Modern Chemistry (Gendai Kagaku). Dec., 34-38, 1985].

Throughout the specification and the claim, the fused proteins represented by the amino acid sequence C where the X in the sequence is 1) and 2) above are called Thβ₄-AM1 and Thβ₄-AM2, respectively.

(1) <u>Process for the production Of TNF$_{AM1}$ and TNF$_{AM2}$</u>

The captioned two proteins may be produced in a conventional manner where the DNA encoding the corresponding amino acid sequence is incorporated into an appropriate vector DNA, and the resulting rDNA is used to trasform host organisms including animal cells, yeasts, B. subtilis, E. coli and other microorganisms to induce the expression.

In order to incorporate a DNA encoding the amino acid sequence of TNF$_{AM1}$ or TNF$_{AM2}$ into a vector DNA in an expressible manner, as is well known, the DNA may be incorporated downstream of the Shine-Dalgarno sequence (hereunder referred to as the SD sequence) of a vector DNA possessing a promotor sequence (being usually downstream of an operator sequence) and the SD sequence which is located downstream of the promotor sequence.

Alternatively, first the DNA may be incorporated into a vector DNA, and then a promotor sequence (usually together with an operator sequence) and the SD sequence may be inserted upstream thereof.

Processes for expression of genetic information of an exogenous gene by techniques using recombinant DNA are described generally in "Techniques for utilizing gene recombinant (4)", 1983, Science Forum; "Molecular Cloning", 1982, Cold Spring Harbor Lab.; "Introduction into cells and expression of recombinant genes", 1983, Kyoritsu Shuppan Cor.; etc.

The case where E. coli is used as the host will be illustrated in a Reference Example.

Alternatively, in case yeast is used as the host, the genetic information may be expressed as described hereunder.

Plasmid vector pMA56 with a promotor for alcohol dehydrogenase (ADHI) incorporated therein ("Nature", 298, pp. 347-350, 1982) has an EcoRI site downstream of the promotor. Thus, the DNA encoding the amino acid sequence of TNF$_{AM1}$ or TNF$_{AM2}$ may be recovered as BamHI/PstI fragment from, for example, a recombinant described in a Reference Example, and then may be inserted into pMA56 at the EcoRI site downstream of the ADHI promotor thereof using EcoRI/BamHI linker and PstI/EcoRI linker to be controlled by the ADHI promotor, thereby expressing the genetic information in yeast.

In addition, because repressible acidic phosphatase (PH05) promotor-having pAM82 ("Proc. Natl. Acad. Sci. U.S.A.", 80, PP. 1-5. 1983) has an XhoI site downstream of the PH05 promotor, the DNA encoding the amino acid sequence of TNF$_{AM1}$ or TNF$_{AM2}$ may be recovered as a BamHI/PstI fragment from, for example, a recombinant described in a Reference Example, and then may be inserted into pMA82 at its XhoI site downstream of the PH05 promotor thereof using BamHI/XhoI linker and PstI/XhoI linker to be controlled by PH05 promotor, thereby making the expression of the genetic information possible in yeast.

B. subtilis may also be employed as the host as follows to express the genetic information of the DNA encoding the amino acid sequence of TNF$_{AM1}$ or TNF$_{AM2}$.

pTUB285 having an $\alpha$-amylase promotor which is originally possessed by B. subtilis Marburs strain ("Gene", 34, p. 148, 1985) has a HincII site downstream of the promotor and a signal peptide. Thus, the DNA encoding the amino acid sequence of TNF$_{AM1}$ or TNF$_{AM2}$ may be recovered as BamHI/PstI fragment from, for example, a recombinant described in a Reference Example, and then may be inserted into pTUB285 at its HincII site using HincII/BamHI linker and HincII/PstI linker which conform to the amino acid frame of the signal peptide to be controlled by the $\alpha$-amylase promotor thereby enabling the expression of the genetic information in B. subtilis.

(2) Process for the separation and purification of TNF$_{AM1}$ and TNF$_{AM2}$

First, the transformed host cells are collected by, for example, centrifugation, and then crushed by treatment with ultrasonic waves or lysozyme. Here a hypotonic solution may be used, and in some cases coexistence of a surfactant such as SDS or a protein-denaturating agent such as guanidine HCl may produce a better result.

The crushed cell-containing solution is subjected to centrifugation to provide a supernatant.

The thus-prepared supernatant containing TNF$_{AM1}$ or TNF$_{AM2}$ may be purified according to any conventional method of purifying proteins.

That is, the supernatant may be subjected to purification by ion exchange chromatography using a basic anion exchanger, salting out, dialysis, gel filtration, hydrophobic chromatography, high performance molecular sieve chromatography, electrophoresis, etc., in the given order or by any desired combination of these methods.

The basic anion exchanger is preferred to be DEAE-Sephadex A-25 or A-50, DEAE-Sepharose CL-6B, or DEAE-Sephamil (all made by Pharmacia AB), but any other diethylamino, aminoethyl, or quaternary-aminoethyl group-containing anion exchanger may be used.

Preferable embodiments of the buffer solution available for use include a Tris-HCl or phosphate buffer solution at pH 6.0 - 9.0. Any of these buffer solutions may be used at a low concentration of about 0.05 M to dilute the culture containing the object polypeptide to a saline concentration of 0.1 M or less, and then the resulting solution is contacted with an anion exchanger which adsorbs TNF$_{AM1}$ or TNF$_{AM2}$.

The elution of TNF$_{AM1}$ or TNF$_{AM2}$ is carried out with a saline solution containing 0.1-0.2 M of NaCl or KCl. The polypeptide is eluted at a saline concentration of about 0.2 M. The contact with the anion exchanger is preferably conducted by a column process, but a batch process may be employed if the contact is conducted on a large scale.

Before the anion exchange chromatography is carried out, the solution is preferably pre-treated with an ultrafiltration membrane to remove lower molecular materials, thereby improving the purification efficiency.

The solution resulting from the anion exchange chromatography is subjected to dialysis and concentration followed by gel filtration. Embodiments of carriers for the gel filtration include Sephadex G-75 and G-100 (manufactured by Pharmacia AB), Sephacryl S-200 (manufactured by Pharmacia AB), Biogel P-100 (manufactured by Biorad), and Toyo Pearl HW-50 and HW-55 (manufactured by Toyo Soda Corp.). The buffer solution intended for use in the gel filtration may be a Tris-HCl or phosphate buffer solution at a pH of 6.0 - 9.0. To prevent adsorption it is desired that 0.2 - 0.5 M of a salt such as NaCl be added to the solution.

Alternatively, the TNF$_{AM1}$ or TNF$_{AM2}$ resulting from the anion exchange chromatography may be purified by hydrophobic chromatography. Here, Butyl-Toyo Pearl 650 (manufactured by Toyo Soda Corp.) or the like may be used as the carrier, and a salt such as ammonium sulfate or NaCl is employed to elute the TNF$_{AM1}$ or TNF$_{AM2}$.

The TNF$_{AM1}$ or TNF$_{AM2}$-containing solution purified by gel filtration or hydrophobic chromatography is then subjected to fast protein exchange chromatography using a Pharmacia FPLC (Fast Protein, Peptide, Polynucleotide, Liquid Chromatography) system on Mono Q HR5/5 column (a high performance anion exchanger column manufactured by Pharmacia AB) to provide a purified sample.

The conditions for the fast protein anion exchange chromatography are the same as for the ion exchange chromatography using a carrier such as DESE-Sepharose mentioned previously.

(3) Process for the production of $Th\beta_4$-AM1 or AM2

The captioned polypeptides may be prepared by combining DNA encoding the amino acid sequence of $Th\beta_4$ with another DNA encoding the sequence: x-(the amino acid sequence corresponding to the base sequence constructed by linking guanine with the beginning of the base sequence of the fourth exon of TNF-$\alpha$) through an Asp-Pro linkage and then inducing the expression of the resulting rDNA in the same manner as described in the above

The rDNA may be prepared in any of the following three manners:

a) DNA encoding (the amino acid sequence of $Th\beta_4$)-Asp-Pro is linked with DNA encoding x-(the amino acid sequence corresponding to the base sequence constructed by linking guanine with the beginning of the base sequence of the fourth exon of TNF-$\alpha$);

b) DNA encoding (the amino acid sequence of $Th\beta_4$) is linked with DNA encoding Asp-Pro-x-(the amino acid sequence corresponding to the base sequence constructed by linking guanine with the beginning of the base sequence of the fourth exon of TNF-$\alpha$);

or

c) DNA encoding (the amino acid sequence of $Th\beta_4$), DNA encoding Asp-Pro and DNA encoding x-(the amino acid sequence corresponding to the base sequence constructed by linking guanine with the beginning of the base sequence of the fourth exon of TNF-$\alpha$) are linked with one another simultaneously in their order.

An example of above a) will be explained herebelow.

PHH58, which is a cDNA complementary to one of the mRNAs synthesized in the course of differentiation of malignant bone marrow into monocytes by stimulation with exogenous differentiation inducing agents (Biochemical and Biophysical Research Communications, vol. 132, No. 1, pp. 100-109, 1985), is treated with PstI followed by recovery of the produced DNA fragment of about 530 bp. Then the fragment is treated with HinfI to give a PstI/HinfI fragment of about 220 bp and a HinfI/PstI fragment of about 310 bp. The former PstI/ HinfI fragment is cleaved with MnℓI (Bairabos Inc., New England in USA) to give a 127 bp MnℓI/HinfI fragment which is then linked with the above HinfI/PstI fragment to construct a MnℓI/PstI fragment encoding the amino acid sequence of $Th\beta_4$. The resulting construction is linked with the MnℓI/PstI fragment of plasmid vector pUC540 to give plasmid pUC540Th$\beta_4$ of about 3.5 kbp.

pUC540 is a product of cloning of EcoRI/BamHI fragment from plasmid pDR540 having a tac promotor inserted into plasmid vector pUC8 at the EcoRI/BamHI cleavage site; the two plasmids are commercially available from Pharmacia.

Then the pUC540Th$\beta_4$ is subjected to treatments involving linking with a BamHI linker to provide a DNA encoding both the amino acid sequence of $Th\beta_4$ and the amino acid sequence Asp-Pro which becomes the linkage connecting the former sequence to x-(the amino acid sequence corresponding to the base sequence constructed by linking guanine with the beginning of the base sequence of the fourth exon of TNF-$\alpha$).

The DNA encoding x-(the amino acid sequence corresponding to the base sequence constructed by linking guanine with the beginning of the base sequence of the fourth exon of TNF-$\alpha$) may be synthesized chemically essentially according to the process described in "Nucleic Acids Res.", 10, pp. 7439-7448, 1981, "Biochemistry", 17, pp. 1257-1267, 1978, or elsewhere.

For example, pUC540AM1 (corres. to the case where x represents the 1) referred to above) and pUC540AM2 (corres. to the case where x represents the 2) referred to above) disclosed in Example 2 of Japanese patent application disclosure No. 62-282587 (Japanese patent application No. 61-169522) may be treated with NcoI (Japan Gene Inc.), Mung Bean nuclease and PstI to provide the object DNAs encoding x-(the amino acid sequence corresponding to the base sequence constructed by linking guanine with the beginning of the base sequence of the fourth exon of TNF-$\alpha$). This is because in those plasmids the DNA fragments encoding x-(the amino acid sequence corresponding to the base sequence constructed by linking guanine with the beginning of the base sequence of the fourth exon of TNF-$\alpha$) is linked with the Tac promotor downstream the BamHI cleavage site, and the starting codon ATG, which is followed by an amino acid the first codon of which is G, exists immediately after the BamHI cleavage site. An embodiment of the case where pUC540AM1 is used will be explained in detail in a Reference Example.

(4) Process for the separation and purification of $Th\beta_4$-AM1 and AM2

The captioned polypeptides may be separated and purified in the same manner as described in the above (2).

The standard single dose of TNF-$\alpha$, TNF$_{AM1}$, TNF$_{AM2}$, $Th\beta_4$ -AM1 or $Th\beta_4$-AM2 or a mixture thereof according to the present invention is $1 \times 10^8$ units (corres. to about 1 mg) for adults. The single dose of the respective polypeptides or their mixture is dissolved in 1 mℓ of a physiological saline solution or encapsulated in liposome for controlled release and then administered intravenously or via another route. Here, it should be understood that the above specified dose is only for reference, and actually the doctor in charge of the respective patients will of course determine the dose in view of their ages, conditions, etc. Ranges may include 0.01 - 10 mg.

The $LD_{50}$ of $TNF_{AM1}$ to mice is $9.5 \times 10^7$ units/kg (Baℓb/c), $2.7 \times 10^7$ units/kg (C3H/He) and $5.8 \times 10^7$ units/kg (C57BL/6), and that of $TNF_{AM2}$ to mice is $2.9 \times 10^7$ units/kg (Baℓb/c), $2.7 \times 10^7$ units/kg (C3H/He) and $2.7 \times 10^7$ units/kg (C57BL/6). These values are very large as compared with those of TNF-$\alpha$ which are $1.0 \times 10^7$ units/kg to Baℓb/c mice, $7.8 \times 10^6$ units/kg to C3H/He mice and $1.5 \times 10^7$ units/kg to C57BL/6. Thus, clinically the former two polypeptides may be evaluated to be much useful than TNF-$\alpha$. The $LD_{50}$ of the fused proteins was almost the same as that of TNF-$\alpha$.

Hereunder, the present invention will be explained in more detail with reference to working examples, etc. where the levels of the respective samples (units/mℓ) were determined as follows on the basis of the cytotoxicity to L-929 cells ("Proceeding of National Academy Science of U.S.A.", 72, pp. 3666 - 3670 using the level of TNF-$\alpha$ provided by its maker Asahi Kasei Co. in Japan as the reference standard.

### Cytotoxicity to L-929 cells

L-929 cells are cultured in Eagles' Minimum Essential Medium (hereunder referred to only as MEM) with 5 % foetal calf serum (hereunder referred to only as FCS) added thereto until 100 mℓ of the medium contains $8 \times 10^4$ cells, and then the cells are grown in a flat-bottomed plate having 96 wells.

The growth conditions are 2 hours at $37°C$ in the presence of 5 % $CO_2$, and 100 % $H_2O$, and the procedures may be the same as far the conventional cell culture. Then actinomycin D is added to the medium to a final concentration of 1 $\mu$g/mℓ, and the volume of the culture solution is adjusted to 150 mℓ. Immediately thereafter 50 $\mu$ℓ of the sample diluted appropriately with MEM medium is added to the culture solution. Here, $ED_{50}$ may be determined by adjusting the dilution appropriately.

The L-929 cells having a final volume of 200 $\mu$ℓ are cultured for an additional 18 hours under the same conditions as described above.

In order to determine the cell necrosis activity, first the whole medium is removed followed by addition of a 2 % methyl alcoholic solution containing 0.2 % crystal violet for fixation staining. Crystal violet stains all the eukaryotic cells, but does not stain those cells left in the bottom of the flask as the result of necrosis; so the cell necrosis activity may be determined directly. The staining degree is measured on the basis of adsorption at $OD_{590nm}$, and is compared with that of a control to determine the cell necrosis activity. This activity is defined as follows.

The dilution of the sample which allows the survival of 50 % of L-929 cells (N) is determined. Rabbit TNS is used as the control, and its activity n (units/mℓ) is determined using $2.4 \times 10^6$ units/mg/mℓ of TNF-$\alpha$. The dilution which provides $ED_{50}$ of rabbit TNS is determined (C).

The activity of the sample (units/mℓ) is calculated by the equation N/C x n.

### Reference Example 1 Production, separation and purification of $TNF_{AM1}$

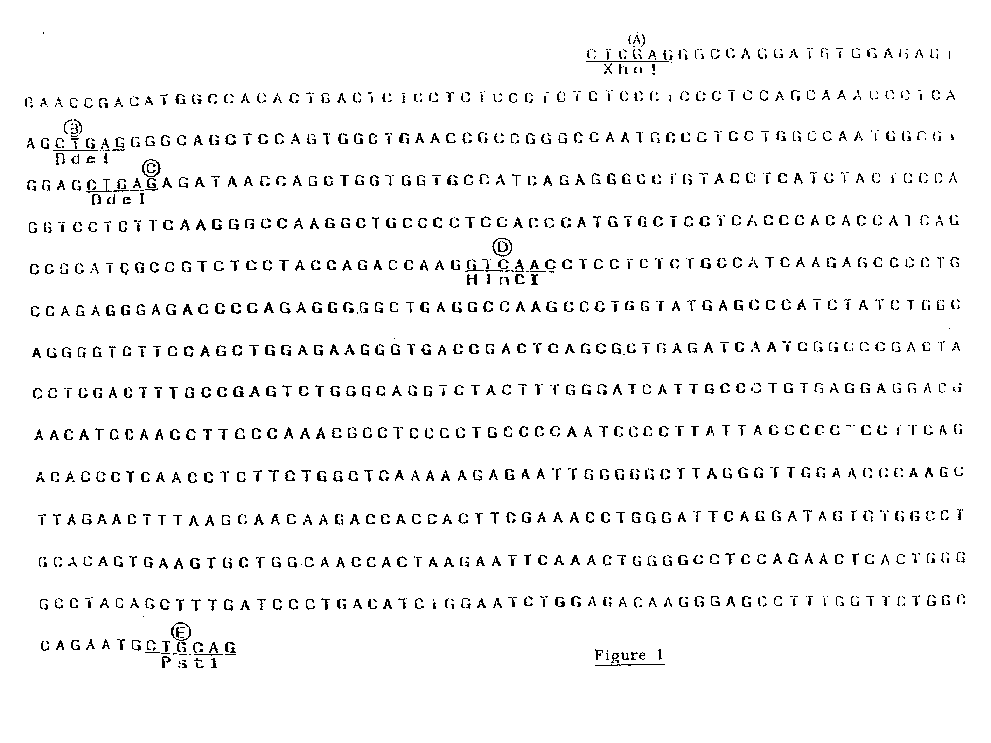

1) The genome gene of the anti-tumor polypeptide extracted from human acute monocytic leukemia cell THP-1 (Japanese patent application disclosure No. 62-282587) was cleaved with XhoI and PstI to recover the XhoI/PstI fragment shown in Fig. 1. Then this fragment was inserted into plasmid vector pUC540, which has Tac promotor and SD sequence, at the Saℓl/PstI site to prepare plasmid pUC540$^{TNF}$x/p.

2) Separately, the XhoI/PstI fragment shown in Fig. 1 was cleaved with HincII to recover 294 bp XHoI/HincII fragment and 521 bp HincII/PstI fragment. Further the 294 bp fragment was partially cleaved with DdeI to recover 206 bp DdeI/HincII fragment.

The thus-recovered 521 bp HincII/PstI fragment and 206 bp DdeI/HincII fragment were combined with 72/73 bp double-strand DNA synthesized with a DNA synthesizer type 380A (ABI Corp. in U.S.A.) and shown in Table 1 given below followed by insertion into the pUC540$^{TNF}$x/p at the BamHI/PstI site to provide pUC540$^{TNF}$ AM1.

## Table 1

### Base sequence of synthetic DNA (73/72 bp)

MetValArgSerSerThrArgThrProSerArgLysProVal

5'-GATCCATGGTTAGAAGCTCCACCCGTACCCCGAGCCGTAAACCGGTA
GTACCAATCTTCGAGGTGGGCATGGGGCTCGGCATTTGGCCAT

AlaHisValValAlaAsnProGln

GCCCATGTTGTAGCAAACCCTCAAGC
CGGGTACAACATCGTTTGGGAGTTCGACT

3) To 100 $\mu$g of pUC540$^{TNF}$AM1 there was added 120 units of Ncol followed by culture at 37°C overnight. The completion of the digestion was confirmed by agar gel electrophoresis followed by extraction with phenol and then with chloroform and purification of the DNA on Sephadex G50 column.

4) To the DNA there was added 90 units of Mung Been nuclease and the mixture was allowed to stand at 37°C for 10 minutes. Then the mixture was heated at 70°C for 5 minutes to inactivate the nuclease.

5) The mixture was cultured at 37°C overnight after addition of 160 units of Pstl. Then 5% acrylamide gel was used to recover 770 bp DNA (about 1 $\mu$g).

6) E. coli JM103 with the DNA from the above 5) incorporated therein was subcultivated in a 1 x YT medium (0.8% bactotrypton + 0.5% bactoyeast extracts + 0.5% NaCl) containing 50 $\mu$g/ml of ampicillin at 37°C, and then transferred, in a proportion of 1%, to a 500 ml Sakaguchi flask containing 100 ml of a 1 x YT medium with 50 $\mu$g/ml of ampicillin added thereto. The mixture was cultured in the same manner at 37°C. When the $OD_{660}$ reached 0.3, isopropyl $\beta$-D-thiogalactopyranoside was added to the culture to a final concentration of 0.7 mM followed by further culture for twenty-four hours. The thus-obtained E. coli was collected with a centrifuge, washed with 1 x PBS (0.8 % NaCl + 0.02% KCl + 0.02% KH$_2$PO$_4$ + 0.115% Na$_2$HPO$_4$), and suspended in 10 ml of 1 x PBS, followed by application of ultrasonic waves to the culture to crush the E. coli cells. Then the polypeptide expressed by the DNA was separated and purified in a conventional manner. This polypeptide has an amino acid sequence shown below, and its molecular weight was 17500 ± 500 (SDS polyacrylamide electrophoresis).

Val-Arg-Ser-Ser-Thr-Arg-Thr-Pro-Ser-Arg-Lys-Pro-Val-Ala-His-Val-Val-(the amino acid sequence corresponding to the base sequence constructed by linking guanine with the beginning of the base sequence of the fourth exon of TNF-$\alpha$)

Reference Example 2 Production, separation and purification of TNF$_{AM2}$

Following the procedures of 1) to 5) In Reference Example 1, but substituting the base sequence encoding Cys for that encoding Ser which is the fifth amino acid encoded by the synthetic DNA shown in Table 1 in Reference Example 1, there was obtained another rDNA. This recombinant was incorporated in E. coli JM103 and treated in the same manner as in Reference Example 1 to separate and purify TNF$_{AM2}$ expressed by said DNA. This polypeptide has an amino acid sequence shown below, and its molecular weight was 17500 ± 500 (SDS polyacrylamide electrophoresis).

Val-Arg-Ser-Cys-Thr-Arg-Thr-Pro-Ser-Arg-Lys-Pro-Val-Ala-His-Val-Val-(the amino acid sequence corresponding to the base sequence constructed by linking guanine with the beginning of the base sequence of the fourth exon of TNF-$\alpha$)

Reference Example 3 Production, separation and purification of Th$\beta$4-AM1, AM2

1) One hundred $\mu$g of pHH58 was digested with 250 units of Pstl at 37°C to separate off Pstl/Pstl

fragment of 530 bp using 1.2% agarose gel. The fragment was extracted from the gel followed by purification on a hydroxy apatite column to recover 2.7 μg of DNA.

2) Then the DNA was digested with 12 units of Hinfl to effect the respective recovery of $^{5'}$PstI/Hinfl$^{3'}$ fragment of 220 bp and $^{5'}$Hinfl/PstI$^{3'}$ fragment of 310 bp using 8% acrylamide gel. The recovery was carried out in a conventional manner using a DEAE cellulose column after the extraction of the two fragments from the gel. Their detection based on UV absorption (OD260 nm) was believed impossible to effect, so the quantity of the two fragments recovered was estimated according to the following equation on the assumption that 100% was recovered.

Quantity of PstI/Hinfl fragment recovered

= 2.7 x 220 / (310 + 220) a 1.1 μg

Quantity of Hinfl/PstI fragment recovered

= 2.7 x 310 / (310 + 220) = 1.6 μg

3) The PstI/Hinfl fragment was digested with 8 units of MnℓI, and a MnℓI/Hinfl fragment of 127 bp was separated and recovered using 8% acrylamide gel; the yield was about 100 - 150 ng.

4) Twenty six ng of the MnℓI/Hinfl fragment was mixed with 65 ng of Hinfl/PstI fragment followed by addition of 87 units of T4DNA ligase, and the mixture was reacted at 16°C for 1 hour. Then the enzyme was inactivated at 70°C for 5 minutes followed by addition of 15 units of PstI and a two hour culture at 37°C to give about 90 ng of DNA.

5) The DNA from the above 4) was mixed with the BamHI/PstI fragment of pUC540 (26ng) in the ethanol (final concentration: 70%) in the presence of 100mM NaCℓ. The precipitate was recovered and dissolved in 5 uℓ of distilled water and then subjected to freeze-drying to about 0.5 μℓ to remove the ethanol and the distilled water. Thereafter 0.4 μℓ of 10 x ligase reaction buffer solution (10mM ATP, 660mM Tris-HCℓ (pH 7.6), 66mM magnesium chloride and 50mM dithiothreithol) and 35 units of T4DNA ligase were added to the residue followed by culture at 4°C overnight to provide about 3.5 kbp of pUC540Thβ4.

6) One hundred μg of pUC540Thβ4 was digested with 190 units of Hinfl at 37°C overnight, and 50 μg of the digestion product was mixed with Klenov fragment of DNA polymerase I (40 units) in the presence of 80 mM of deoxy ATP and 80 μM of deoxy TTP followed by culture at room temperature for 30 minutes.

7) The Klenov fragment mentioned above was inactivated by heating at 70°C for 5 minutes, 120 units of BamHI was added to the culture solution followed by culture at 37°C for 3 hours.

8) The BamHI and Hinfl sites were repaired to flat ends with A and T using 8% acryl gel, and about 450 ng of 135 bp of DNA was separated and recovered using a DEAE cellulose column.

9) Twenty seven ng of the DNA from the above 8) was mixed with 1.6 ng of BamHI linker in the presence of 87 units of T4DNA ligase followed by culture at 4°C overnight to yield DNA encoding (the amino acid sequence of Thβ4 )-Asp-Pro.

10) The DNA from the above 9) (28.6 ng) and the other DNA obtained at the end of the procedures of 1) to 5) in Reference Example 1 were cultured at 4°C overnight together in the presence of 87 units of T4DNA ligase. Then the ligase was inactivated by heating at 70°C for 10 minutes.

11) A twelve hour culture was effected at 37°C after addition of 0.8 units of PstI, and the mixture was heated at 70°C for 10 minutes to inactivate the PstI. Then the mixture was mixed with the BamHI/PstI fragment of plasmid vector pUC540 (76 μg) followed by culture at 4°C overnight in the presence of 90 units of T4DNA ligase. Thus there was provided plasmid pUC540 (Thβ4-AM1).

12) E. coli DH-1 with the plasmid from the above 11) incorporated therein was subjected to shaking culture at 37°C overnight in 10 mℓ of NZY medium (prepared by mixing 5 g of NaCℓ. 2 g of MgCℓ2 .7H2O, 10 g of NZ amine A manufactured by Wako Jun-yaku in Japan and 5 g of yeast extracts in distilled water in a quantity to prepare 1 ℓ of medium) containing 50 μg/mℓ of ampicillin and 10 μg/mℓ of kanamycin. Ten mℓ of the resulting culture solution was added to 100 mℓ of NZY medium containing 50 μg/mℓ of ampicillin and 10 μg/mℓ of kanamycin followed by shaking culture at 37°C for 6 hours. Then all the culture solution (about 100 mℓ) was added to 1 ℓ of NZY medium containing 0.7 mM of isopropyl β-D-galactopyranoside, 50 μg/mℓ of ampicillin and 10 μg/mℓ of kanamycin followed by shaking culture at 37°C overnight.

13) The thus cultured E. coli was collected with a centrifuge (5,000 rpm, 10 minutes) and suspended in 100 mℓ of PBS(-) (Nissui Corp.). The resulting suspension was subjected to centrifugation (5,000 rpm, 10 minutes), and the precipitate was suspended in 1 mM of phenylmethylsulfonyl fluoride (a protease inhibitor). The resulting cell suspension was divided into 10 mℓ portions to which ultrasonic waves were applied three times at graduation 40 of a Branson sonifier for 6 minutes to crush the E. coli cells.

14) The mixture was subjected to centrifugation at 10,000 rpm for 1 hour to recover the supernatant.

15) Ammonium sulfate fractions (60% saturation) from the above supernatant were separated off and subjected to centrifugation at 10,000 rpm for 30 minutes to provide precipitate which was then dissolved in 5 mℓ of PBS(-) followed by dialysis against 5 ℓ of a solution containing 10 mM of Tris-HCℓ (pH 7.4) and 10 mM of NaCℓ. Then the solution was treated by centrifugation at 13,000 rpm overnight to remove the insolubles followed by recovery of the supernatant.

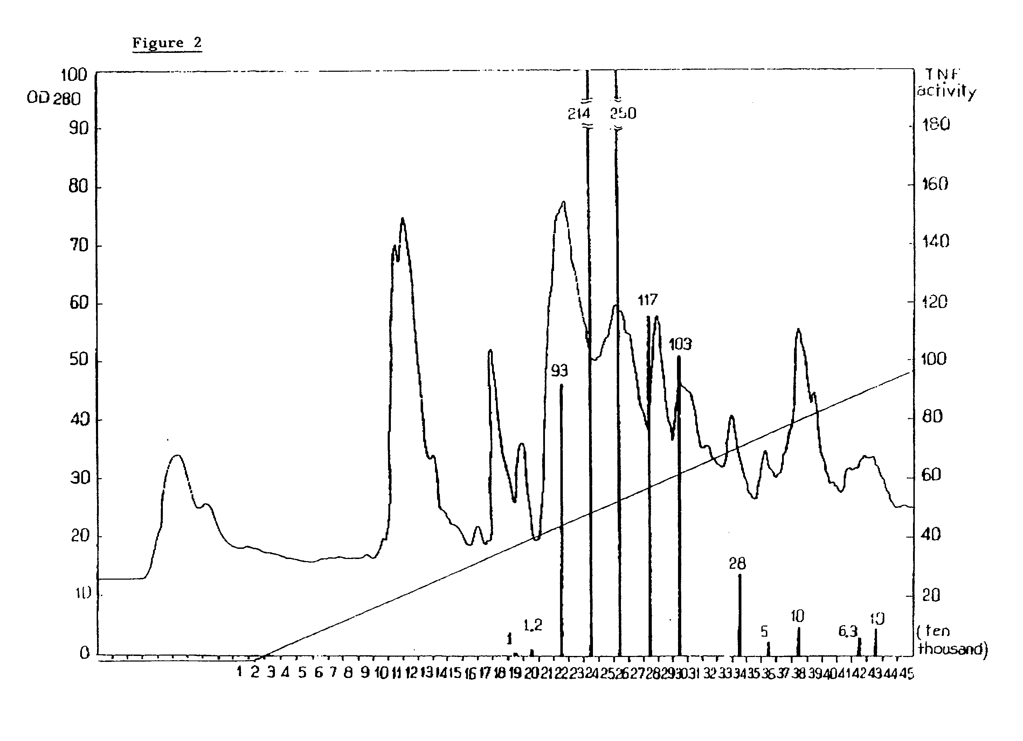

16) The supernatant was subjected to chromatography on Pharmacia Mono Q FPLC column. The flow rate was adjusted to 2.0 mℓ/min., and the 20 mℓ portions were collected in a gradient-elution manner wherein the NaCℓ concentration was increased linearly from 0.01 M to 1 M. The elution pattern is shown in Fig. 2.

In Fig. 2, the consecutive numbers along the axis of abscissa represent the respective fractions, the curve traces the quantity of the protein eluted, and the linear shows the salt content of the eluates (0.01 M to 1 M).

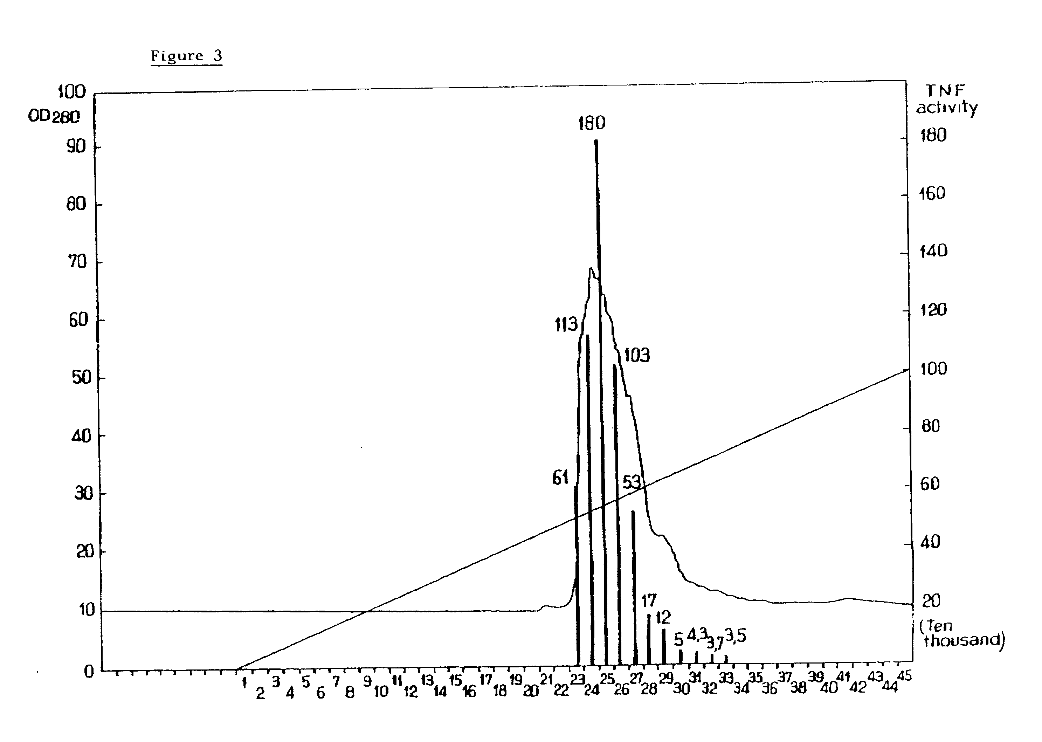

17) Some active fractions Nos. 23-27, which are rich in the protein having the structure of Th$\beta_4$-AM1 were collected and subjected again to the treatment on a Mono Q column to provide 7,5 mℓ of fraction showing only one peak (purity is over 95%). The elution pattern is shown in Fig. 3.

In Fig. 3, all the consecutive numbers along the axis of abscissa, the curve and the linear are defined in the same manner as in Fig. 2.

The study by gel filtration using a Pharmacia Superose 12 column revealed that the molecular weight is 67,000 dalton or more, and this value suggested that the molecule is a trimer or tetramer. SDS electrophoresis showed that the molecular weight is 22,000 dalton.

18) Following the procedures described in the above 1) t0 17), but substituting the DNA obtained in Reference Example 2 for that obtained at the end of the procedures of 1) to 5) in Reference Example 1 used in the above 10), there was obtained Th$\beta_4$-AM2. Its production was confirmed by the Maxam-Gilbert's method to determine the corresponding base sequence of the resulting fused protein up to CGT corresponding to an amino acid Arg which is the the tenth amino acid from the terminal of the amino acid sequence of the Th$\beta$4-linkage, and is peculiar to ThB4-AM2.

Example 1

A solution for intravenous injection was prepared by dissolving 1 x 10$^6$ units (about 1 mg) of TNF-$\alpha$, TNF$_{AM1}$, TNF$_{AM2}$, Th$\beta_4$-AM1 or Th$\beta_4$-AM2 or a mixture thereof in 1 mℓ of a physiological saline solution.

Example 2

A slow-release preparation was prepared by dispersing and encapsulating 1 x 10$^6$ units (about 1 mg) of TNF-$\alpha$, TNF$_{AM1}$, TNF$_{AM2}$, Th$\beta_4$-AM1 or Th$\beta_4$-AM2 or a mixture thereof into liposome.

Example 3

A solution was prepared by dissolving 1 x 10$^6$ units (about 1 mg) of TNF-$\alpha$, TNF$_{AM1}$, TNF$_{AM2}$, Th$\beta_4$-AM1 or Th$\beta_4$-AM2 or a mixture thereof in 1 mℓ of a physiological saline solution. The resulting solution may be administered intravenously with 3 x 10$^6$ units of $\gamma$-INF.

Example 4

A slow-release preparation was prepared by dispersing and encapsulating 1 x 10$^6$ units (about 1 mg) of TNF-$\alpha$, TNF$_{AM1}$, TNF$_{AM2}$, Th$\beta_4$-AM1 or Th$\beta_4$-AM2 or a mixture thereof into liposome. The resulting preparation may be administered with 3 x 10$^6$ units of $\gamma$-INF.

Example 5

A solution was prepared by dissolving 1 x 10$^6$ units (about 1 mg) of TNF-$\alpha$, TNF$_{AM1}$, TNF$_{AM2}$, Th$\beta_4$-AM1 or Th$\beta_4$-AM2 or a mixture thereof in 1 mℓ of a physiological saline solution. The resulting solution may be administered intravenously with an appropriate amount of AZT.

Example 6

A slow-release preparation was prepared by dispersing and encapsulating 1 x $10^6$ units (about 1 mg) of TNF-$\alpha$, $TNF_{AM1}$, $TNF_{AM2}$, Th$\beta_4$-AM1 or Th$\beta_4$-AM2 or a mixture thereof into liposome. The resulting preparation may be administered with an appropriate amount of AZT.

Example 7

A solution wad prepared by dissolving 1 x $10^6$ units (about 1 mg) of TNF-$\alpha$, $TNF_{AM1}$, $TNF_{AM2}$, Th$\beta_4$-AM1 or Th$\beta_4$-AM2 or a mixture thereof in 1 ml of a physiological saline solution. The resulting solution may be administered intravenously with 3 x $10^6$ units of $\gamma$-INF and an appropriate amount of AZT.

Example 8

A solution was prepared by dissolving 1 x $10^6$ units (about 1 mg) of TNF-$\alpha$, $TNF_{AM1}$, $TNF_{AM2}$, Th$\beta_4$-AM1 or Th$\beta_4$-AM2 or a mixture thereof into liposome. The resulting preparation may be administered with 3 x $10^6$ units of $\gamma$-INF and an appropriate amount of AZT.

Experiment 1 Anti-HIV effects of TNF-$\alpha$, $TNF_{AM1}$ and $TNF_{AM2}$

Molt-4 cells which belong to T-cell line positive to human T-cell leukemia virus type I (Minowada J. et al., "Rosette forming human lymphoid cell lines 1. Establishment and evidence for origin of thumus-derived lymphocytes" described in J. Natl. Cancer Inst. 49, 891, 1972) were infected with HIV-1 at a duplication of 0.002. 90% or more of the cells were positive to the HIV antigen. Hereunder these infected cells will be referred to as Molt-4/HIV cells.

These cells were transferred to PRMI-1640 (Nissui Corp.) supplemented with 10% of foetal calf serum, penicillin (100 IU/ml) and streptomycin (100 $\mu$g/ml) at 37°C for 4 days subculture. Then 1 x $10^5$/ml of the cells were treated with 0, $10^{-1}$, 10 or $10^3$ ng/ml of the respective samples, and the cell survival rates were calculated according to the methylene blue method 1, 2, 3 or 4 days after the treatment. The results are shown in Tables 2 - 4 where the values are number of the cells (x $10^4$/ml), and the bracketed numbers are cell survival rates. The cell survival rate immediately after the administration of the samples was 96% in all the cases.

## Table 2: Effects of TNF-α

| Level | Days after treatment | | | |
|---|---|---|---|---|
| | 1 day | 2 days | 3 days | 4 days |
| $10^3$ | 14(78) | 10(47) | 5(22) | 1( 2) |
| 10 | 22(78) | 10(56) | 6(38) | 3(25) |
| $10^{-1}$ | 20(97) | 35(97) | 75(97) | 180(96) |
| 0 | 19(95) | 42(96) | 75(97) | 182(97) |

## Table 3: Effects of TNF$_{AM1}$

| Level | Days after treatment | | | |
|---|---|---|---|---|
| | 1 day | 2 days | 3 days | 4 days |
| $10^3$ | 12(74) | 9(47) | 4(22) | 1( 2) |
| 10 | 19(80) | 10(56) | 7(40) | 3(23) |
| $10^{-1}$ | 19(98) | 35(98) | 72(95) | 180(95) |
| 0 | 20(97) | 42(97) | 74(96) | 178(95) |

## Table 4: Effects of TNF$_{AM2}$

| Level | Days after treatment | | | |
|---|---|---|---|---|
| | 1 day | 2 days | 3 days | 4 days |
| $10^3$ | 13(77) | 9(45) | 3(21) | 1( 2) |
| 10 | 20(80) | 11(58) | 6(39) | 3(23) |
| $10^{-1}$ | 19(97) | 33(97) | 73(97) | 178(95) |
| 0 | 21(97) | 44(98) | 75(97) | 182(96) |

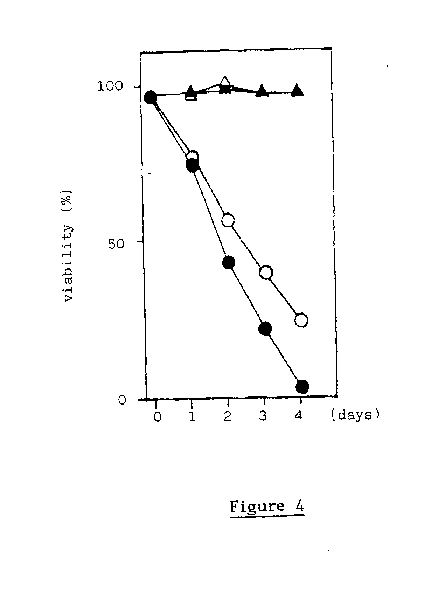

Fig. 4 represents the effects given in Table 4 graphically. In Fig. 4, the abscissae represent the days after the treatment with the samples, and the ordinates represent the cell survival rates (%) in the respective cases of the samples, and △ and ▲ ○ and ● show the results at the level of 0, $10^{-1}$, 10, $10^3$ ng/m$\ell$, respectively. Here the data of △ and ▲ overlap each other.

As is clearly shown in Tables 2 - 4, the survival rates of the Molt-4/HIV cells drastically decrease by the administration of 10 - $10^3$ ng/m$\ell$ of TNF-α, TNF$_{AM1}$ or TNF$_{AM2}$, and the degree of the decrease was almost the same in all the cases.

Experiment 2 Anti-HIV effects of Th$\beta_4$-AM1 and Th$\beta_4$-AM2

In th same manner as in Experiment 1, but using the samples at the level of 0, 1, 10 or $10^2$ ng/m$\ell$, the effects of Th$\beta_4$-AM1 and Th$\beta_4$-AM2 on Molt-4 cells and Jurkat cells (Schneider U. et al., "Characterization of EBV-genome negative "null" and "T" cell lines derived from children with acute lymphoblastic leukemia and leukemic transformed non-Hodgkin lymphoma" described in Int. J. Cancer, 19. 621, 1977) infected with or not infected with HIV were determined. The results are shown in Tables 5 - 8 using a cell survival rate as the indication.

### Table 5: Effects of Th$\beta_4$-AM1 on Molt-4/HIV cells

| Level | Days after treatment | | | |
| | 1 day | 2 days | 3 days | 4 days |
|---|---|---|---|---|
| $10^2$ | 90 | 67 | 27 | 3 |
| 10 | 95 | 85 | 43 | 23 |
| 1 | 95 | 94 | 88 | 75 |
| 0 | 98 | 97 | 98 | 95 |

The survival rate immediately after the administration was 98% in all the cases.

### Table 6: Effects of Th$\beta_4$-AM1 on Jurkat/HIV cells

| Level | Days after treatment | | | |
| | 1 day | 2 days | 3 days | 4 days |
|---|---|---|---|---|
| $10^2$ | 97 | 49 | 22 | 9 |
| 10 | 95 | 63 | 17 | 18 |
| 1 | 98 | 81 | 58 | 60 |
| 0 | 98 | 94 | 95 | 95 |

The survival rate immediately after the administration was 96% in all the cases.

## Table 7: Effects of Thβ₁-AM2 on Molt-4/HIV cells

| Level | Days after treatment | | | |
|---|---|---|---|---|
| | 1 day | 2 days | 3 days | 4 days |
| $10^2$ | 92 | 68 | 30 | 4 |
| 10 | 97 | 84 | 45 | 22 |
| 1 | 96 | 95 | 90 | 74 |
| 0 | 98 | 97 | 97 | 95 |

The survival rate immediately after the administration was 98% in all the cases.

## Table 8: Effects of Thβ₁-AM2 on Jurkat/HIV cells

| Level | Days after treatment | | | |
|---|---|---|---|---|
| | 1 day | 2 days | 3 days | 4 days |
| $10^2$ | 97 | 51 | 23 | 8 |
| 10 | 95 | 64 | 16 | 17 |
| 1 | 97 | 80 | 59 | 59 |
| 0 | 98 | 95 | 94 | 95 |

The survival rate immediately after the administration was 96% in all the cases.

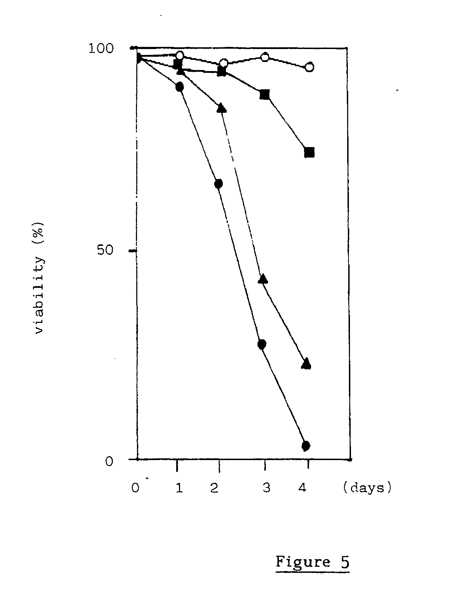

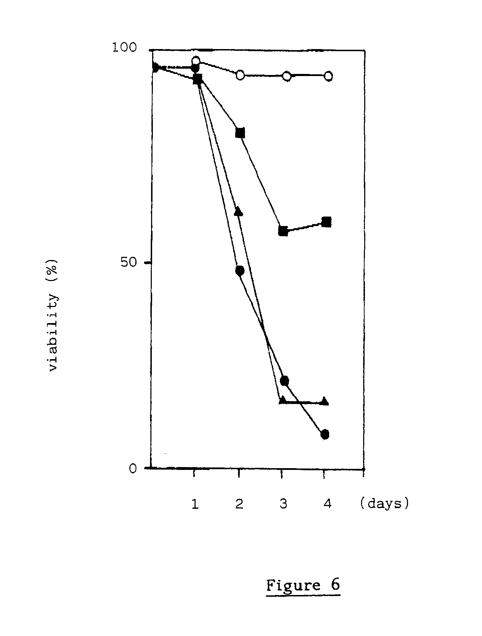

Figs. 5 - 6 represent the effects given in Tables 5 - 6 graphically. In Figs. 5 - 6, the abscissae represent the days after the treatment with the samples, and the ordinates represent the cell survival rates (%) in the respective cases of the samples, and O, ■, ▲, ● show the results at the level of 0, 1, 10, $10^2$ ng/mℓ, respectively.

As is clearly shown in the tables and the graphs, the survival rates of the HIV-infected cells drastically decreased at the level of 10 ng or $10^2$ ng/mℓ, and the degree of the decrease was almost the same in all the cases.

Experiment 3 Anti-HIV effects of TNF$_{AM2}$ (2)

In th same manner as in Experiment 1, but using the samples at the level of 0, $10^{-1}$, 1, 10 or $10^2$ ng/mℓ, the effects of TNF$_{AM2}$ on CCRF-CEM cells (Forley, G. E. et al., "Continuous culture of human lymphoblasts from peripheral blood of a child with acute leukemia" described in Cancer, 18, 522, 1965), H9 cells (Popovic M. et al., "Detection, isolation, and continuous production of cytopathic retroviruses (HTLV-III) from patients with AIDS and pre-AIDS" described in Science, 224, 497, 1984), Jurkat cells and Molt-4 cells infected with or not infected with HIV were determined. More particularly, the living cells were calculated 3 days after the administration of the samples, and then the culture media were diluted by being mixed with fresh media containing the same level of the respective samples at a rate of 1:4, and the living cells were calculated again 3 days after the dilution. The results are shown in Tables 9 - 24 in terms of living rates and growth rates (relative growth rates). The growth rates were calculated according to the following equation:

$$\frac{\text{Number of the living cells in the presence of TNF}_{\alpha M2}}{\text{Number of the living cells in the absence of TNF}_{\alpha M2}} \times 100$$

Table 9: Effects on CCRF-CEM/HIV cells observed 3 days after the administration

| Level | $10^2$ | 10 | 1 | $10^{-1}$ | 0 |
|---|---|---|---|---|---|
| Living rate | 88 | 87 | 97 | 97 | 99 |
| Growth rate | 43 | 66 | 81 | 95 | |

Table 10: Effects on CCRF-CEM cells observed 3 days after the administration

| Level | $10^2$ | 10 | 1 | $10^{-1}$ | 0 |
|---|---|---|---|---|---|
| Living rate | 88 | 87 | 97 | 97 | 99 |
| Growth rate | 43 | 66 | 81 | 95 | |

Table 11: Effects on H9/HIV cells observed 3 days after the administration

| Level | $10^2$ | 10 | 1 | $10^{-1}$ | 0 |
|---|---|---|---|---|---|
| Living rate | 78 | 88 | 91 | 99 | 97 |
| Growth rate | 0.72 | 0.82 | 1.03 | 0.99 | |

Table 12: Effects on H9 cells observed 3 days after the administration

| Level | $10^2$ | 10 | 1 | $10^{-1}$ | 0 |
|---|---|---|---|---|---|
| Living rate | 88 | 94 | 91 | 94 | 95 |
| Growth rate | 0.88 | 0.89 | 0.97 | 0.94 | |

**Table 13: Effects on Jurkat/HIV cells observed 3 days after the administration**

| Level | $10^2$ | 10 | 1 | $10^{-1}$ | 0 |
|---|---|---|---|---|---|
| Living rate | 80 | 85 | 96 | 94 | 96 |
| Growth rate | 0.71 | 0.86 | 0.97 | 1.03 | |

**Table 14: Effects on Jurkat cells observed 3 days after the administration**

| Level | $10^2$ | 10 | 1 | $10^{-1}$ | 0 |
|---|---|---|---|---|---|
| Living rate | 91 | 93 | 94 | 96 | 95 |
| Growth rate | 0.97 | 0.96 | 0.96 | 0.94 | |

**Table 15: Effects on Molt-4/HIV cells observed 3 days after the administration**

| Level | $10^2$ | 10 | 1 | $10^{-1}$ | 0 |
|---|---|---|---|---|---|
| Living rate | 16 | 26 | 86 | 96 | 97 |
| Growth rate | 0.02 | 0.03 | 0.51 | 0.98 | |

**Table 16: Effects on Molt-4 cells observed 3 days after the administration**

| Level | $10^2$ | 10 | 1 | $10^{-1}$ | 0 |
|---|---|---|---|---|---|
| Living rate | 93 | 90 | 94 | 98 | 99 |
| Growth rate | 0.71 | 0.81 | 0.95 | 0.92 | |

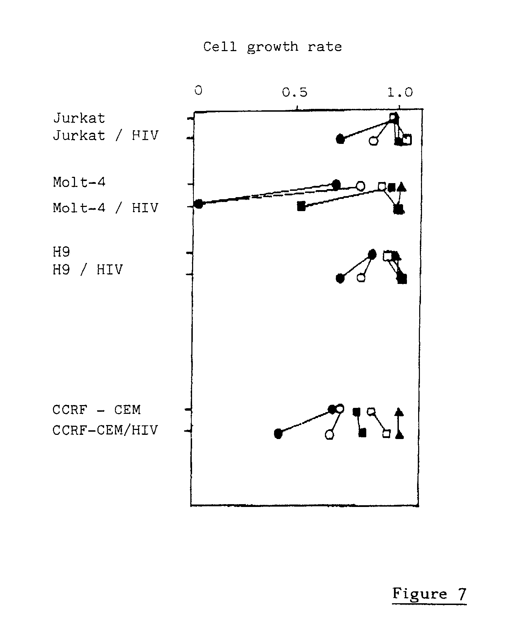

Fig. 7 represents the growth rates reported in Table 9 - 16 graphically. In Fig. 7, ▲, □, ■, ○ and ● show the results at the level of 0, $10^{-1}$, 1, 10, $10^2$ ng/m$\ell$, respectively.

At the level of 10 ng/m$\ell$ or more, the difference in cytotoxicity between HIV-infected and non-HIV-infected cells was remarkably in any cells, and thus the excellent selective cytotoxicity of $TNF_{AM2}$ to the HIV-infected cells was established.

Table 17: Effects on CCRF-CEM/HIV cells observed 6 days after the administration

| Level | $10^2$ | 10 | 1 | $10^{-1}$ | 0 |
|---|---|---|---|---|---|
| Living rate | 77 | 84 | 92 | 96 | 97 |
| Growth rate | 0.27 | 0.50 | 0.84 | 0.93 | |

Table 18: Effects on CCRF-CEM cells observed 6 days after the administration

| Level | $10^2$ | 10 | 1 | $10^{-1}$ | 0 |
|---|---|---|---|---|---|
| Living rate | 92 | 92 | 97 | 96 | 98 |
| Growth rate | 0.71 | 0.78 | 0.89 | 0.90 | |

Table 19: Effects on H9/HIV cells observed 6 days after the administration

| Level | $10^2$ | 10 | 1 | $10^{-1}$ | 0 |
|---|---|---|---|---|---|
| Living rate | 81 | 91 | 92 | 93 | 93 |
| Growth rate | 0.36 | 0.53 | 0.88 | 0.94 | |

Table 20: Effects on H9 cells observed

6 days after the administration

| Level | $10^2$ | 10 | 1 | $10^{-1}$ | 0 |
|---|---|---|---|---|---|
| Living rate | 89 | 93 | 92 | 95 | 95 |
| Growth rate | 0.66 | 0.68 | 0.84 | 0.94 | |

Table 21: Effects on Jurkat/HIV cells observed 6 days

after the administration

| Level | $10^2$ | 10 | 1 | $10^{-1}$ | 0 |
|---|---|---|---|---|---|
| Living rate | 36 | 43 | 83 | 87 | 90 |
| Growth rate | 0.11 | 0.17 | 0.50 | 0.94 | |

Table 22: Effects on Jurkat cells observed 6 days

after the administration

| Level | $10^2$ | 10 | 1 | $10^{-1}$ | 0 |
|---|---|---|---|---|---|
| Living rate | 39 | 73 | 86 | 92 | 91 |
| Growth rate | 0.20 | 0.48 | 0.82 | 0.98 | |

Table 23: Effects on Molt-4/HIV cells observed 6 days

after the administration

| Level | $10^2$ | 10 | 1 | $10^{-1}$ | 0 |
|---|---|---|---|---|---|
| Living rate | 0 | 0 | 45 | 97 | 99 |
| Growth rate | 0 | 0 | 0.12 | 1.03 | |

## Table 24: Effects on Molt-4 cells observed 6 days

## after the administration

| Level | $10^2$ | 10 | 1 | $10^{-1}$ | 0 |
|---|---|---|---|---|---|
| Living rate | 92 | 95 | 97 | 95 | 96 |
| Growth rate | 0.71 | 0.81 | 0.95 | 0.92 | |

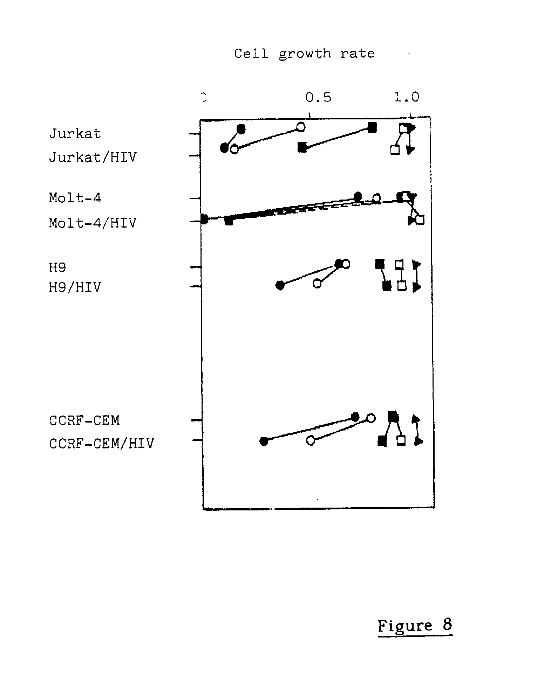

Fig. 8 represents the growth rates reported in Table 17 - 24 graphically, In Fig 8, ▲, □, ■, ○ and ● show the results at the level of 0, $10^{-1}$, 1, 10, $10^2$ ng/mℓ, respectively.

At the level of 10 ng/mℓ or more, the difference in cytotoxicity between HIV-infected and non-HIV-infected cells was remarkably for any cells, and thus the excellent selective cytotoxicity of $TNF_{AM2}$ to the HIV-infected cells was established.

Experiment 4 Cytotoxicity to non-HIV-infected cells

The results shown in Experiments 1 - 3 given above clearly demonstrate that TNF-$\alpha$, $TNF_{AM1}$, $TNF_{AM2}$, Th$\beta_4$-AM1 and Th$\beta_4$-AM2 have excellent cytotoxicity to HIV-infected cells. In addition, the results of Experiment 3 confirm that $TNF_{AM2}$ has no substantial cytotoxicity to non-HIV-infected cells.

In order to study the details of the cytotoxicity of the various samples mentioned above to HIV-infected cells, the sensitivity of the above samples to non-HIV-infected cells was determined by calculating the cell growth rates using an absorbance at $OD_{630nm}$ as the indication according to the methylene blue dying method. The results are shown in Tables 25 - 26. The tested samples are as follows:

Sample 1: TNF-$\alpha$ Sample 2: $TNF_{AM2}$ Sample 3: Th$\beta_4$-AM1

Table 25: Measurements in CCRF-CEM cells observed 10 days after the administration

| Sample No. | Quantity (units/mℓ) | | | | |
|---|---|---|---|---|---|
| | 1 | 10 | $10^2$ | $10^3$ | $10^4$ |
| 1 | 0.94 | 0.92 | 0.92 | 0.44 | 0.37 |
| 2 | 0.95 | 0.90 | 0.95 | 0.96 | 0.91 |
| 3 | 1.02 | 1.06 | 0.98 | 0.95 | 0.96 |

Table 26: Measurements in Molt-4 cells observed 12 days after the administration

| Sample No. | Quantity (units/mℓ) | | | | |
|---|---|---|---|---|---|
| | 1 | 10 | $10^2$ | $10^3$ | $10^4$ |
| 1 | 0.90 | 0.92 | 0.82 | 0.37 | 0.52 |
| 2 | 1.00 | 0.96 | 0.93 | 0.92 | 0.88 |
| 3 | 0.98 | 0.94 | 0.90 | 1.04 | 0.94 |

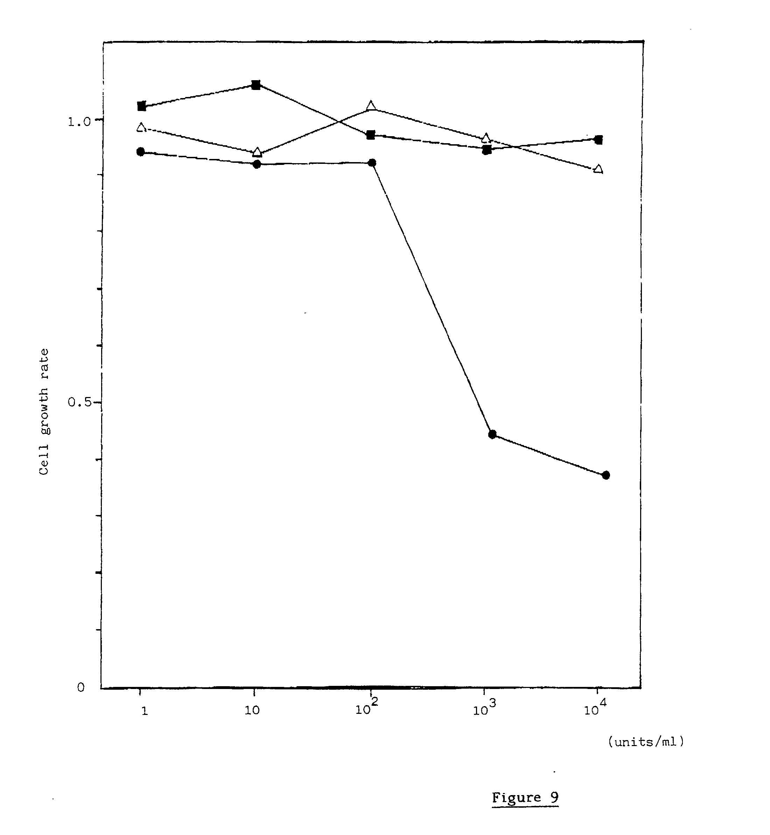

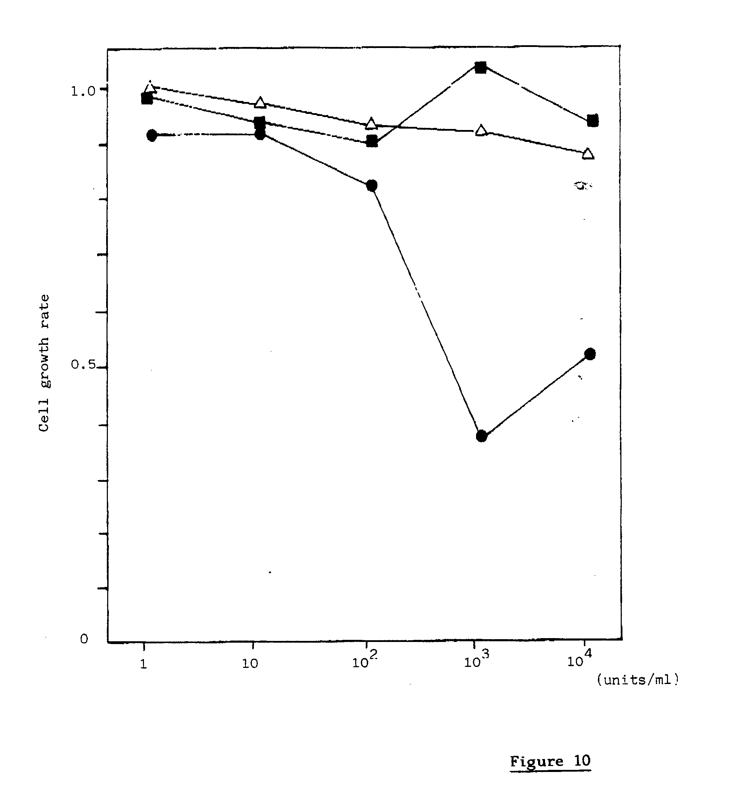

Figs. 9 - 10 show the results reported in Tables 25 - 26 graphically. In the figures, ●, Δ and ■ represent the data of TNF-α, $TNF_{AM2}$ and Thβ4-AM1, respectively.

From the foregoing results, TNF-α may be appreciated to have almost the same level of cytotoxicity to HIV-infected cells as the other samples. But, more importantly, its cytotoxicity to non-HIV-infected cells is very high as compared with those of the other samples. Thus, it is believed that TNF-α has low selective cytotoxicity to HIV-infected cells, and therefore its usefulness as a medicine is evaluated to be lower than the other samples tested.

**Claims**

1. An anti-AIDS preparation comprising, as an active agent, a pharmaceutically effective amount of a polypeptide selected from the group consisting of TNF-α, $TNF_{AM1}$ $TNF_{AM2}$, Thβ4-AM1, Thβ4-AM2, represented by the amino acid sequences given below, respectively, and mixtures of at least two of these polypeptides, wherein:

   $TNF_{AM1}$: Met-Val-Arg-Ser-Ser-Thr-Arg-Thr-Pro-Ser-Arg-Lys-Pro-Val-Ala-His-Val-Val-(the amino acid sequence corresponding to the base sequence constructed by linking guanine with the beginning of the base sequence of the fourth exon of TNF-α) $TNF_{AM2}$: Met-Val-Arg-Ser-Cys-Thr-Arg-Thr-Pro-Ser-Arg-Lys-Pro-Val-Ala-His-Val-Val-(the amino acid sequence corresponding to the base sequence constructed by linking guanine with the beginning of the base sequence of the fourth exon of TNF-α)

Ser-Asp-Lys-Pro-Asp-Met-Ala-Glu-Ile-Glu-Lys-Phe-Asp-

Lys-Ser-Lys-Leu-Lys-Lys-Thr-Glu-Thr-Gln-Glu-Lys-Asn-Pro-Leu-Pro-

Ser-Lys-Glu-Thr-Ile-Glu-Gln-Glu-Lys-Gln-Ala-Gly-Glu-Ser-Asp-Pro-

Val-Arg-Ser-Ser-Thr-Arg-Thr-Pro-Ser-Arg-Lys-Pro-Val-Ala-His-Val-

Val-

Th$\beta_4$-AM1: (the amino acid sequence corresponding to the base sequence constructed by linking guanine with the beginning of the base sequence of the fourth exon of TNF-$\alpha$)

Ser-Asp-Lys-Pro-Asp-Met-Ala-Glu-Ile-Glu-Lys-Phe-Asp-

Lys-Ser-Lys-Leu-Lys-Lys-Thr-Glu-Thr-Gln-Glu-Lys-Asn-Pro-Leu-Pro-

Ser-Lys-Glu-Thr-Ile-Glu-Gln-Glu-Lys-Gln-Ala-Gly-Glu-Ser-Asp-Pro-

Val-Arg-Ser-Cys-Thr-Arg-Thr-Pro-Ser-Arg-Lys-Pro-Val-Ala-His-Val-

Val-

Th$\beta_4$-AM2: (the amino acid sequence corresponding to the base sequence constructed by linking guanine with the beginning of the base sequence of the fourth exon of TNF-$\alpha$).

2. The preparation of claim 1, further comprising a pharmaceutically acceptable carrier.

3. A composition for treating AIDS in a mammal infected with HIV retroviruses, comprising the preparation of claims 1 or 2.

4. A composition for preventing the reoccurence of AIDS in a mammal displaying AIDS symptoms and killing substantially all T4 cells of said mammal infected with HIV retrovirus, comprising the preparation of claims 1 or 2.

EP 0 450 240 A1

(A)
CTCGAGGGCCAGGATGTGGAGAGT
Xho I

GAACCGACATGGCCACACTGACTCTCCTCTTCCTTCTCTCCCTCCCTCCAGCAAACCCTCA

AGCTGAGGGGCAGCTCCAGTGGCTGAACCGCCGGGCCAATGCCCTCCTGGCCAATGGCGT
(a)
Dde I

GGAGCTGAGAGATAACCAGCTGGTGGTGCCATCAGAGGGCCTGTACCTCATCTACTCCCA
(C)
Dde I

GGTCCTCTTCAAGGGCCAAGGCTGCCCCTCGACCCATGTGCTCCTCACCCACACCATCAG

CCGCATCGCCGTCTCCTACCAGACCAAGGTCAACCTCCTCTCTGCCATCAAGAGCCCCTG
(D)
HincI

CCAGAGGGAGACCCCAGAGGGGGCTGAGGCCAAGCCCTGGTATGAGCCCATCTATCTGGG

AGGGGTCTTCCAGCTGGAGAAGGGTGACCGACTCAGCGCTGAGATCAATCGGCCCGACTA

CCTCGACTTTGCCGAGTCTGGGCAGGTCTACTTTGGGATCATTGCCCTGTGAGGAGGACG

AACATCCAACCTTCCCAAACGCCTCCCCTGCCCCAATCCCCTTATTACCCCCTCCTTCAG

ACACCCTCAACCTCTTCTGGCTCAAAAAGAGAATTGGGGGCTTAGGGTTGGAACCCAAGC

TTAGAACTTTAAGCAACAAGACCACCACTTCGAAACCTGGGATTCAGGATAGTGTGGCCT

GCACAGTGAAGTGCTGGCAACCACTAAGAATTCAAACTGGGGCCTCCAGAACTCACTGGG

GCCTACAGCTTTGATCCCTGACATCTGGAATCTGGAGACAAGGGAGCCTTTGGTTCTGGC

CAGAATGCTGCAG
(E)
Pst I

Figure 1

Figure 2

Figure 3

Figure 4

Figure 5

Figure 6

Figure 7

Figure 8

Figure 9

Figure 10

European
Patent Office

**EUROPEAN SEARCH
REPORT**

Application Number

EP 90 40 0938

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CANCER CELLS vol. 1, no. 2, October 1989, pages 62 - 63; TRACEY K.J et al: "The role of cachectin/TNF in AIDS" * the whole document * | 1-3 | A 61 K 37/02 A 61 K 37/24 |
| X | JOURNAL OF IMMUNOLOGY vol. 140, 1988, BALTIMORE pages 120 - 124; WONG G.H.W. et al: "IN VITRO anti human immunodeficiency virus activities of tumor necrosis factor alpha and interferon gamma" * the whole document * | 1 | |
| X | JOURNAL OF VIROLOGY vol. 63, no. 6, June 1989, pages 2504 - 2509; MATSUYAMA T. et al: "Cytcidal effect of TNF on cells chronically infected with human immunodeficiency virus(HIV):Enhancement of HIV Replication" * the whole document * | 1 | |
| A | JOURNAL OF BIOLOGICAL RESPONSE MODIFIERS vol. 7, 1988, Raven Press.ltd. NEW YORK pages 587 - 595; SOMA G.I. et al: "Biological activities of novel rTNF having N-terminal amino acid sequences derived from cytotoxic factors produced by THP1 cells" * the whole document * | 1 | TECHNICAL FIELDS SEARCHED (Int. Cl.5) |
| A | EP-A-0 341 100 (MIZUNO,DEN' ICHI ET SOMA;GEN-ICHIRO) * the whole document * | 1 | A 61 K C 07 K C 12 N |

The present search report has been drawn up for all claims

| Place of search | Date of completion of search | Examiner |
|---|---|---|
| The Hague | 21 December 90 | LE CORNEC N.D.R. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document